(19) Europäisches Patentamt
European Patent Office
Office européen des brevets

(11) Publication number: **0 459 373 A2**

(12) # EUROPEAN PATENT APPLICATION

(21) Application number: **91108636.1**

(22) Date of filing: **28.05.91**

(51) Int. Cl.[5]: **C08F 20/04**, C08F 2/06, A61K 9/32, A61K 9/58

(30) Priority: **31.05.90 US 531276**

(43) Date of publication of application: **04.12.91 Bulletin 91/49**

(84) Designated Contracting States: **AT BE CH DE DK ES FR GB GR IT LI LU NL SE**

(71) Applicant: **THE B.F. GOODRICH COMPANY**
**3925 Embassy Parkway**
**Akron Ohio 44313-1799(US)**

(72) Inventor: **Hsu, Chin Chien**
**160 Williamsburg Drive**
**Avon Lake, Ohio 44012(US)**
Inventor: **Lochhead, Robert Yeats**
**32705 Greenwood Court**
**Avon Lake, Ohio 44012(US)**
Inventor: **Masler, William Frank**
**1026 Mattingly Road**
**Hinckley, Ohio 44233(US)**
Inventor: **Sehm, Eugene Joseph**
**1104 Portage Lakes Drive**
**Akron, Ohio 44319(US)**

(74) Representative: **von Kreisler, Alek et al**
**Patentanwälte von Kreisler Selting Werner**
**Deichmannhaus am Hauptbahnhof**
**W-5000 Köln 1(DE)**

(54) **Bioadhesive carboxylic polymer composition and method relating thereto.**

(57) The present invention relates generally to polymeric based compositions, particularly adhesive and most particularly bioadhesive polymeric compositions, capable of adhering to a substrate as a lubricant, slow release medium, or the like. More particularly, the polymeric adhesive composition of the present invention is directed to acrylic-type polymer and copolymer compositions polymerized in a substantially non-aqueous medium to provide a material having an average particle size (without mechanical grinding) less than about 10 microns.

EP 0 459 373 A2

## Field of The Invention

The present invention relates generally to bioadhesive polymeric compositions which can adhere to a biosurface and provide long term moisturizing, lubrication or the like; in certain embodiments, the bioadhesive material of this invention may be advantageously used as a stable medium for slowly releasing an active agent at a particular location on a biological or similar-type substrate. More specifically, the polymeric bioadhesive composition of the present invention is directed to acrylic-type polymer and copolymer compositions polymerized in a substantially non-aqueous medium to provide a bioadhesive material with an average particle size diameter (without mechanical grinding) of less than about 10 microns.

## Background of the Invention

Carboxylic-type homopolymers and copolymers are known generally as water swellable agents or additives. Such polymers have a significant commercial presence as thickeners for any one of a large number of commercial products. Numerous patents are directed to such compositions, including U.S. Pat. Nos. 2,798,053, 3,074,852, 3,202,577, 3,330,729, 3,915,921, 3,940,351, 4,062,817, 4,066,583, 4,226,848, 4,267,103 and 4,758,641.

Although the above references do teach carboxylic-type polymers polymerized in aqueous and non-aqueous systems. none of these references teach an adhesive composition polymerized in a non-aqueous medium to an average particle size of less than about 10 microns to provide a material having an appropriate mucilage viscosity such that the end product is useful as a bioadhesive lubricant, slow release agent, or the like.

U.S. 4,615,697 to Robinson, entitled "BIOADHESIVE COMPOSITIONS AND METHODS OF TREATMENT THEREWITH", teaches polymerization in an aqueous medium. The Robinson patent and the subsequent continuation thereof, U.S. 4,795,436, do not teach a bioadhesive composition polymerized in a non-aqueous medium to an average particle size of less than about 10 microns without grinding.

## Summary of the Invention

The present invention is directed to a hydrophilic, water insoluble, carboxylic polymeric bioadhesive type material useful as a biolubricant, medicinal carrier medium or the like. The bioadhesive is preferably manufactured according to the following process:

charging a reactor continuously or in a single step with a solution comprising a non-aqueous solvent system and a monomer charge dissolved therein, said monomer charge comprising carboxylic monomer having at least one activated carbon-to-carbon olefinic double bond, ad at least one carboxyl group, wherein the amount of carboxyl containing monomer is in the range of about 5% to 100% by weight of the monomer charge and wherein the balance of the monomer charge comprises vinylidene comonomer;

polymerizing the reagent system into a polymerization product in a closed vessel in an inert atmosphere under autogenous pressure or in an open vessel under reflux at atmospheric pressure,

maintaining a temperature of polymerization in the range of about 0 C to about 100 C, wherein the solubility of the polymerization product causes the polymerization product to precipitate out of solution having an average particle size of less than about 10 microns in diameter.

## DETAILED DESCRIPTION OF THE PREFERRED EMBODIMENT

The preferred embodiment of the present invention is directed to hydrophilic, water insoluble, carboxylic-type polymeric materials, such as polycarbophil (polyacrylic acid cross-linked with divinyl glycol) or other polycarboxylic acid- type polymer or copolymer materials, cross-linked or not, which have an average particle size of less than about 10 microns in diameter and which have a mucilage viscosity sufficient to provide a bioadhesive lubricant, slow release agent, or the like. These polymers are polymerized in a solvent system other than water and are obtained by means of a precipitation polymerization, wherein as the polymerization progresses, the solubility of the polymerized product decreases and will precipitate out at a molecule size sufficient to create an average particle size less than about 10 microns in diameter without grinding.

The preferred polymers of the present invention are carboxyl containing polymers having molecular weights greater than about 500 to several million, more preferably greater than about 10,000 to 1,000,000. The bioadhesive material of the present invention preferably has a Brookfield mucilage viscosity in a 1 wt % aqueous solution of greater than about 10,000 cps measured at 20 rpm.

The carboxylic monomers useful in the production of the polymers of this invention are the olefinically-unsaturated carboxylic acids containing at least one activated carbon-to-carbon olefinic double bond, and at least one carboxyl group. The acid contains at least one olefinic double bond which readily functions in a polymerization, due to its presence in the monomer molecule, either in the alpha-beta position with respect to a carboxyl group, i.e., -C = C-COOH, or as part of a terminal methylene grouping, i.e., $CH_2$ = C<.

The presence of a terminal methylene grouping in a carboxylic monomer makes this type of compound much more polymerizable than where the double bond is intermediate in the carbon structure. Olefinically-unsaturated acids of this class include such materials as the acrylic acids typified by the acrylic acid itself, methacrylic acid, ethacrylic acid, alpha-chloroacrylic acid, alpha-cyano acrylic acid, beta methylacrylic acid (crotonic acid), alpha phenyl acrylic acid, beta-acryloxy propionic acid, sorbic acid, alpha-chloro sorbic acid, beta-styryl acrylic acid (1-carboxy-4-phenyl butadiene-1,3), itaconic acid, citraconic acid, mesaconic acid, glutaconic acid, aconitic acid maleic acid, fumaric acid, tricarboxy ethylene, and the like. As used herein, the term "carboxylic acid" includes the polycarboxylic acids and those acid anhydrides, such as maleic anhydride, wherein the anhydride group is formed by the elimination of one molecule of water from two carboxyl groups located on the same polycarboxylic acid molecule. Acid anhydrides useful herein include those having the general structure

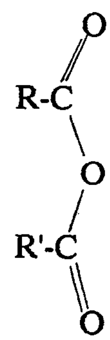

wherein R and R' are selected from the group consisting of hydrogen, halogen and cyanogen (-CN) groups and alkyl, aryl, alkaryl, aralkyl, and cycloalkyl groups such as methyl, ethyl propyl, octyl, decyl, phenyl, tolyl, xylyl, benzyl cyclohexyl, and the like.

The preferred carboxylic monomers for use in the present invention are the monoolefinic acrylic acids having the general structure

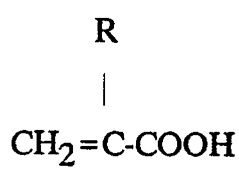

wherein R is a substituent selected from the class consisting of hydrogen, halogen, and the cyanogen groups, monovalent alkyl radicals, monovalent aryl radicals, monovalent aralkyl radicals, monovalent alkaryl radicals and monovalent cylcoaliphatic radicals. Of this class, acrylic and methacrylic acid are most preferred due generally to lower cost, ready availability and an ability to form superior polymers. Another useful carboxylic monomer is maleic anhydride or the acid thereof.

The polymers contemplated include both homopolymeric carboxylic acids or anhydrides thereof, or the defined carboxylic acids copolymerized with one or more other vinylidene monomers containing at least one terminal $CH_2$ = CH< group. Such materials include, for example, acrylic ester monomers including those acrylic ester monomers having long chain aliphatic groups such as derivatives of an acrylic acid represented by the formula

$$\begin{array}{l} \quad\quad\;\; R' \;\; O \\ \quad\quad\;\; | \quad\; \| \\ CH_2{=}C{-}C{-}O{-}R \end{array}$$

wherein R is an alkyl group having from 10 to 30 carbon atoms, preferably 10 to 20 carbon atoms and R' is hydrogen or a methyl or ethyl group, present in the copolymer in amount, for example, from about 1 to 30 weight percent, and for some uses more preferably, about 5 to 15 weight percent. Representative higher alkyl acrylic esters are decyl acrylate, isodecyl methacrylate, lauryl acrylate, stearyl acrylate, biphenyl acrylate and melissyl acrylate and the corresponding methacrylates. Mixtures of two or three or more long chain acrylic esters may be successfully polymerized with one of the carboxylic monomers to provide a material useful in the present invention.

The polymers contemplated also include acrylamides and methacrylamides, particularly where the amide nitrogen is substituted with a $C_1$ - $C_{30}$ alkyl or aryl substituent to provide secondary and tertiary substituted acrylamides and methacrylamides.

The polymers of the present invention may be cross-linked with any polyfunctional vinylidene monomer containing at least 2 terminal CH2 = C< groups, including for example, butadiene, isoprene, divinyl benzene, divinyl naphthalene, allyl acrylates and the like. A particularly useful cross-linking monomer is divinyl glycol. Other suitable cross linking agents include polyalkenyl polyethers having more than one alkenyl ether grouping per molecule. Preferred crosslinkers have alkenyl groups in which an olefinic double bond is present attached to a terminal methylene grouping, $CH_2 = C<$. They are made by etherification of a polyhydric alcohol containing at least 4 carbon atoms and at least 3 hydroxyl groups.

Compounds of this class may be produced by reacting an alkenyl halide, such as allyl chloride or allyl bromide with a strongly alkaline aqueous solution of one or more polyhydric alcohols. The product is a complex mixture of polyethers with varying numbers of ether groups. Efficiency of the polyether crosslinking agent increases with the number of potentially polymerizable groups on the molecule. It is preferred to utilize polyethers containing an average of two or more alkenyl ether groupings per molecule.

Other crosslinking monomers include for example, diallyl esters, dimethallyl ethers, allyl or methallyl acrylates and acrylamides, tetra-allyl tin, tetravinyl silane, polyalkenyl methanes, diacrylates and dimethacrylates, divinyl compounds as divinyl benzene, polyallyl phosphate, diallyloxy compounds and phosphite esters and the like. Typical agents are allyl pentaerythritol, allyl sucrose, trimethylolpropane triacrylate, 1,6-hexanediol diacrylate, pentaerythritol triacrylate, tetramethylene dimethacrylate, ethylene diacrylate, ethylene dimethacrylate, triethylene glycol dimethacrylate and the like. Other monomers can also be used, particularly in conjunction with acrylic esters, including the acrylic nitriles, $\alpha,\beta$-olefinically unsaturated nitriles useful in the interpolymers embodied herein are preferably the monoolefinically unsaturated nitriles having from 3 to 10 carbon atoms such as acrylonitrile, methacrylonitrile, ethacrylonitrile, chloroacrylonitrile, and the like. The monomer constituents may contain from 1-30 carbon atoms, including aliphatic, aromatic, and aralkyl; other functional groups may also be present, such as, $-SO_3H$, -COOH, $PO_3H_2$, ketone, and the like.

Useful acrylic amides include monoolefinically unsaturated amides which may be incorporated in the interpolymers of this invention have at least one hydrogen on the amide nitrogen and the olefinic unsaturation is alpha-beta to the carbonyl group. Representative amides include acrylamide, methacrylamide, N-methyl acrylamide, N-t-butyl acrylamide, N-cyclohexyl acrylamide, N-ethyl acrylamide and the like, such as $C_1$ - $C_{30}$ mono- and di-substituted acrylamides. Other acryl amides include N-alkylol amides of alpha, beta-olefinically unsaturated carboxylic acids including those having from 4 to 10 carbon atoms such as N-methylol acrylamide, n-ethanol acrylamide, n-propanol acrylamide, n-methylol methacrylamide, n-ethanol methacrylamide, N-methylol maleimide, N-methylol maleamide, N-methylol maleamic acid, N-methylol maleamide, N-methyl maleamic acid esters, the N-alkylol amides of the vinyl aromatic acids such as N-methylol-p-vinyl benzamide, and the like.

The amount of carboxyl containing monomer is in the range of about 5% to about 100% by weight of the monomer charge, preferably about 50%-100%. The non-carboxyl monomers preferably are vinylidene comonomers, such as alpha olefins containing from 2 to 12 carbon atoms, more preferably from 2 to 8 carbon atoms, such as ethylene, propylene, and the like. Suitable dienes include those containing from 4 to 10 carbon atoms, particularly conjugated dienes as butadiene, isoprene, piperylene and the like. Other useful comonomers include: vinyl esters and allyl esters, such as, vinyl acetate, vinyl benzoate, allyl acetate and the like; vinyl aromatics such as styrene, vinyl toluene, vinyl naphthalene and the like; vinyl and allyl

ethers and ketones such as, vinyl methyl ether, ally methyl ether, vinyl isobutyl ether, and the like; vinyl nitriles such as acrylonitrile, methacrylonitrile and the like; cyanoalkyl acrylates, such as, alpha cyanomethyl acrylate and the like; vinyl halides and vinyl chloride and the like; halovinylates; and divinyls, diacrylates and other polyfunctional monomers, such as, divinyl benzene, divinyl ether, diethylene glycol diacrylate, ethylene glycol dimethacrylate and the like.

Polymerization of the monomer in the solvent medium is usually carried out in the presence of a free radical catalyst in a closed vessel in an inert atmosphere under autogenous pressure or in an open vessel under reflux at atmospheric pressure. Temperature of the polymerization may be varied from about 0°C to about 100°C depending to a degree upon the molecular weight desired in the polymer. Polymerization at 50°C to about 90°C under autogenous pressure using a free radical catalyst is generally effective in producing a polymer yield of 75% to 100%. Typical free radical forming catalysts include peroxygen compounds such as sodium, potassium and ammonium persulfates, caprylyl peroxide, benzoyl peroxide, hydrogen peroxide, pelargonyl peroxide, cumene hydroperoxides, tertiary butyl diperphthalate, tertiary butyl perbenzoate, sodium peracetate, sodium percarbonate, and the like as well as azo-diisobutyronitrile, hereinafter referred to as azoisobutyronitrile. Other catalysts utilizable are the so-called "redox" type of catalyst and the heavy-metal activated catalyst systems. Ultra-violet light may also be used as a source of free radicals. Some systems polymerize solely by heat, but catalysts provide better control. The monomer may be batch charged or continuously added during the course of polymerization or by any other manner of polymerization techniques conventionally used.

If unwanted gelling arises, a feature of the present invention is the neutralization of at least part of the carboxyl groups to prevent such gelling of the polymer with a group 1-A metal compound (such as lithium, sodium, potassium, and cesium) in the form of a hydroxide, oxide, carbonate or the like. Also to prevent gelling, ammonia or an amine can be added, such as morpholine, mono-, di- or triethanolamine, mono-propanolamine, and the like, particularly where the partial polymeric salt is less soluble in the defined medium to high hydrogen bonding solvents than the acid polymer formed. Some exchange to obtain desired salt is also feasible. To prevent unwanted gelling, greater than 1 weight percent of the monomer carboxyl groups are oftentimes neutralized or formed into a salt of the above listed materials. Gelling may or may not be a problem however, depending upon the compatibility of the solvent system with the polymerization product. Generally, where the polymerization product quickly precipitates out of solution, then gelling is generally less of a problem. However depending upon the degree of polymerization desired, the solvent system and monomer system chosen, the amount of neutralization, if any, may have to be determined by ordinary skill and experimentation.

The preferred solvents used are non-aqueous and those normally liquid at room temperature. The polymerization is a precipitation polymerization wherein the monomer system is soluble in the solvent, but as polymerization progresses, the polymer will precipitate out once the molecular weight causes the solubility of the polymer to be outside the range of the solvent.

Critical to the present invention is that the polymer precipitate out having a particle size less than about 10 microns. At such a particle size, it has generally been found that the bioadhesive material will have a smooth feel and will have excellent adherence to bio-surfaces. Furthermore, such a particle size is well suited for retaining active ingredients therein which can be slowly released over time, particularly where the polarity and hydrogen bonding capability of the active ingredient substantially matches the polarity and hydrogen bonding characteristics of the bioadhesive.

In the preferred embodiment, the solvent is selected from acetone, alkyl acetates containing 1 to 6 carbons in the alkyl group, and the like. The most preferred solvent is ethyl acetate, and the preferred monomer charge comprises acrylic acid and salts thereof, a suitable initiator and divinyl glycol (3,4-dihydroxy-1,5-hexadiene) crosslinker. With this preferred solvent and monomer system, neutralization of the monomer carboxyl groups is generally not necessary.

Any of the above described monomers and comonomers could be used as well as any of the above describe solvents and optionally the above described crosslinkers. However, critical to the present invention is that the polymer precipitate out of solution with an average particle size of less than about 10 microns. Preferably, the polymer exhibits a mucilage viscosity of greater than about 50,000 cps based upon a 1 wt % aqueous solution. It would be impossible to specifically describe every possible monomer combination together with all appropriate solvents. Consequently, ordinary skill and experimentation may be necessary to develop any particular embodiment of the present invention.

If a monomer system is selected other than the preferred monomer system described above (acrylic acid and/or salts thereof together with a divinyl glycol crosslinker), an appropriate solvent system must be chosen, so the polymer will precipitate out of solution at a particle size of less than about 10 microns.

If an alternative monomer system is chosen, the solubility of the monomers must be predicted. One

reasonably accurate method of predicting solubility is to determine the solubility parameter value of the monomers. Such values are available from Polymer Handbook, J. Brandsup and E. Immergut, editors, "Solvent Parameter Values" by H. Burrell, 1975, pp. IV-337-359 (hereafter "POLYMER HANDBOOK"). Once the solubility parameter of the monomer system is chosen, then the solubility parameter value of the polymer (to be derived from the monomer system) must then be determined, and such values are also provided in the POLYMER HANDBOOK.

The "solubility parameter" method is a procedure for predicting solubilities of amorphous polymers and, with less justification, crystalline polymers. The value is based upon the free energy change for mixing two pure substances at temperature T, i.e., $\Delta G_{mix} = \Delta H_{mix} - T A S_{mix}$. The solubility parameter method predicts the enthalpy of mixing according to the cohesive energy density, i.e., $E^{coh}$ per unit volume V required to separate completely the molecules in a system (for volatile liquids $E^{coh}$ is usually taken as the energy of vaporization). The solubility parameter is the square root of the cohesive energy density:

$$\delta = (E^{coh}/V)^{1/2} = (E^{\circ}_{vap}/V)^{1/2} = \left[(H^{\circ}_{vap} - RT)/V^{\circ}\right]^{1/2}$$

In this equation, $E^{\circ}_{vap}$ and $H^{\circ}_{vap}$ are the molar energy and enthalpy of vaporization. A connection between $\Delta H_{mix}$ and the solubility parameters of polymer and solvent is made by writing the intermolecular energies in terms of the cohesive energies for pure solvent and undiluted polymer, and therefore:

$$\Delta H_{mix} = n_1 V^{\circ}_1 \phi_2 (\delta_1 - \delta_2)^2$$

wherein $V^{\circ}_1$ is the molar volume of the solvent system, $\phi_2$ is the molar volume fraction of the solute (polymer or monomer system). This equation shows that when the values of the solubility parameters of amorphous polymer and solvent approach one another, $\Delta H_{mix}$ should decrease toward zero. Since the free energy of the mixture decreases with $\Delta H_{mix}$,systems that mix with small heats are expected to have complete miscibility. In applying solubility parameters, miscibility is predicted when $\delta_1$ and $\delta_2$ are within 2 or 3

$$\frac{J^{1/2}}{cm^{3/2}}$$

of one another.

The solubility parameters of most solvents and monomers can be determined from their molar energies or enthalpies of vaporization. High molecular weight polymers however typically cannot be vaporized (they will typically decompose with heating rather than vaporize). Consequently, the solubility parameter for polymers typically must be determined indirectly. Several methods have been employed, most of which use a series of potential solvents with known solubility parameters. In one method, the solubility parameter of the macromolecule is taken as the midpoint of the range of the solubility parameters of those liquids that completely dissolve the polymer. If the polymer is not completely miscible, $\delta_2$ (the solubility parameter of the polymer) is approximated by equating it to the solubility parameter of $\delta_1$ (the solubility parameter of the solvent) of the liquid in which the polymer has the greatest solubility. In another method, the swelling of a lightly cross-linked polymer is measured in the various liquids. The greatest swelling should be found when $\delta_1 \sim \delta_2$. It is also possible to estimate solubility parameters by summing group contributions. The solubility parameters for a large number of polymers and solvents have been compiled, and a rather comprehensive listing can be found in the POLYMER HANDBOOK.

The solubility parameter value for any particular polymer will generally be given as a range according to the solvent system used. The solvent system is generally defined as either strongly, moderately or weakly hydrogen bonding. For each of these solvent types, a solubility parameter range is given for any particular polymer. The solubility parameter range of the polymer preferably should not overlap with the solubility parameter of the monomer system. Preferably, the solubility of parameter of the monomer system is about

25% above the polymer solubility parameter range or about 25% below this range.

Once an appropriate polymer range is found according to the ranges published in the POLYMER HANDBOOK, the handbook will also indicate whether the solvent system must be strongly, moderately or weakly hydrogen bonding. Using this designation as guidance, a solvent system of appropriate hydrogen bonding strength should be selected which has a solubility parameter range encompassing the monomer system. As such, the monomer system is readily soluble in the solvent, and as the polymerization progresses, the polymer product will precipitate out of solution, preferably with an average particle size less than about 10 microns.

If the particle size is too great, a cross-linking agent can be added to diminish the solubility of the polymer in the solvent system, or alternatively, a new solvent can be adopted or an additional solvent component can be added to the solvent system to adjust the solubility of the solvent. Generally, "likes dissolve likes" and therefore to increase the solubility of the polymer in the solvent to thereby cause larger polymer particles to precipitate out, the polarity or hydrogen bonding capability of the solvent should be adjusted to more closely match the polarity or hydrogen bonding capability of the polymer product. Alternatively, if the particle size is too large, the solvent system should be adjusted such that the polarity or hydrogen bonding capability of the polymer is less closely matched with the solvent system, such as by altering the solvent system or modifying the monomer system. Alternatively, the temperature of the system can be altered, wherein increasing the temperature will generally increase the solubility of the polymer and decreasing the temperature generally decreases the solubility of the polymer. As the solubility of the polymer decreases, the particle size generally decreases, and as the solubility of the polymer increases, the particle size generally increases.

Ordinary skill and experimentation may be necessary to find the optimal solvent, monomer system, crosslinker system and/or temperature range for any particular embodiment of the present invention. The particle size should be less than about 10 microns but not so small that the end product exhibits a viscosity so low as to be incapable of acting as a bioadhesive-type material, depending upon the use of the end product.

In the preferred embodiment, polycarbophil and salts thereof are prepared in a solvent selected from acetone, alkyl acetates containing 1 to 6 carbon atoms in the alkyl group in the presence of a suitable initiator and divinyl glycol (3,4-dihydroxy-1,5-hexadiene) crosslinker. Polycarbophil and alkali metal and alkaline earth metal salts thereof, prepared in this manner, have the attributes of bioadhesivity, small particle size without grinding and viscosity of above 50,000 cps when 1% by weight mucilages thereof are measured in water. It is estimated that weight average molecular weight of polycarbophil or crosslinked polyacrylic acid is in the range of about 100,000 to about 10 million, preferably one half million to 5 million.

The polyacrylic acid of this invention is a water-insoluble, crosslinked carboxy-functional polymer that contains specified amounts of carboxyl functionality and crosslinking agent. Suitable monomer for the preferred embodiment of the present invention are acrylic acid and salts thereof.

To prevent gelling of the polymer and to promote discrete particle formation during polymerization, at least a part of the carboxyl groups should be neutralized with a group I-A metal compound in the form of a hydroxide, oxide, or carbonate and the like. Examples of these include sodium, potassium, and the like as a well as reaction with ammonia and certain amines including morpholine, mono-, di- and triethanolamine, mono propanolamine, and other amines where the partial polymeric salt is less soluble in the reaction medium.

Normally, polar and medium to strongly hydrogen bonded solvents cause the free acid containing polymers to gel, and this may or may not be desirable, depending upon the end use application. Solvents suitable in the preferred embodiment are those which are liquid at room temperature of about 22°C selected from acetone and lower alkyl acetates containing 1 to 6, preferably 2 to 4, carbon atoms in the alkyl group. Specfic examples of such acetates include ethyl acetate, isopropyl acetate, n-butyl acetate, and the like. Preferred solvent is ethyl acetate. The amount of solvent used should be such that the monomer solids content is up to about 30% by weight, preferably 10 to 20%.

In general, the amount of water in the solvent should be as low as possible, since if water is allowed to exceed about 3% in the solvent, the reaction mass becomes a substantially solid rubbery mass, and this is generally undesirable for a bioadhesive-type material. Desirable results can be achieved by continuously removing water from the solvent as by passing the solvent through a distillation column or through a bed of a desiccant or a substance which will remove water from the solvent. This problem can be compounded by the fact that the neutralization of the carboxylic acid monomer often produces water as a by-product, depending on the specific neutralizing agent. However, water can be removed and the amount thereof in the reaction mass can be controlled to a level below 3%, preferably .05 to about 1%, in the solvent, in the manner described above.

Polymerization of the acrylic acid monomer or its salt in the solvent medium is usually carried out in the presence of a free radical initiator in a closed vessel in an inert atmosphere and under autogenous pressure, artificially-induced pressure, or in an open vessel under reflux at atmospheric pressure. Temperature of the polymerization may be varied up to about 100° C, preferably about 40 to 80° C, depending on the type of initiator selected. Suitable free radical initiators are those which will convert essentially all of the monomer to polymer at the reaction temperature. Examples of such free radical initiators include di(2-ethylhexyl) peroxydicarbonate, di(sec-butyl) peroxydicarbonate, di(isopropyl) peroxydicarbonate, dicyclohexyl peroxydicarbonate, dicetyl peroxydicarbonate, di(n-propyl) peroxydicarbonate, lauroyl peroxide, and other like peroxides and peroxydicarbonates. The di(2-ethylhexyl) peroxydicarbonate is effective at a reaction temperature of 45 to 55° C whereas lauroyl peroxide is effective at a reaction temperature of 70 to 80° C. Amount of the initiator is generally less than 5%, preferably 0.05 to 2.0%, and especially 0.1 to 1% by weight based on the weight of the monomer charge.

The crosslinker for making polycarbophil is divinyl glycol or 3,4-dihydroxy-1,5-hexadiene. Amount of the crosslinker per 100 weight parts monomer can vary up to about 5% by weight, preferably 0.01 to 3%, especially 0.5 to 2% by weight.

The products of this invention have exceptional bioadhesive properties. The small particle size of the products herein lends itself to better adhesion to mucous membranes and to more extensive applications in non-tablet areas such as lotions, suspensions, gels, syrups and the like. Mix smoothness is exceptionally good and useful in drug and cosmetic applications.

The invention will now be illustrated with a specific example of preparing polycarbophil or polyacrylic acid crosslinked with a small amount of divinyl glycol. The polyacrylic acid product has bioadhesive property and a particle size of less than 10 microns without grinding and its 1% aqueous solution has viscosity exceeding 50,000 cps.

Example

The following ingredients were used in amounts indicated to prepare polycarbophil:

| | |
|---|---|
| Ethyl Acetate Solvent | 1316.42 grams |
| Acrylic Acid Monomer | 180.00 grams |
| Potassium Carbonate | 2.59 grams |
| Divinyl Glycol Crosslinkers | 0.09 grams |
| EHP Initiator | 0.90 grams |
| | 1500.00 grams |

The EHP Initiator was di(2-ethylhexyl) peroxydicarbonate.

A 2-liter jacketed reactor was used in this preparation which reactor had cooling capability and which was provided with a reflux condenser. The reactor was purged with nitrogen to remove moisture and to maintain an inert atmosphere in the reactor. The acrylic acid was preneutralized with anhydrous potassium carbonate by mixing potassium carbonate in acrylic acid until potassium carbonate dissolved in acrylic acid, which took about one-quarter of an hour, while the nitrogen purge was continued. The neutralized acrylic acid was charged to the purged reactor followed by ethyl acetate. The crosslinker was prepared in a 6-dram vial in ethyl acetate and then charged to the reactor. The solution in the reactor was agitated for a few minutes and then agitation was stopped and the nitrogen purge was placed at bottom of reactor and the contents of the reactor were purged with nitrogen for about 20 minutes. With the nitrogen purge at top of reactor again, the initiator was charged to reactor and heating of the reactor was commenced and was continued for 6 hours.

The polymer recovered from the reactor was rotary vacuum dried to remove remaining ethyl acetate solvent. The polymer was particulate and free-flowing with average particle size of less than 10 microns and its Brookfield viscosity of 1% mucilage in water was 59,200 cps measured at 20 rpm. The pH of the mucilage was adjusted to about 7.5. It was also determined that the polymer had the bioadhesive property.

The above discussion has been provided to aid in the understanding of the present invention. Details

provided above are provided primarily to help the ordinary artisan visualize the preferred embodiment and the innumerable other possible embodiments of this invention, and such details are not intended to create any limitations to this invention. Many improvements and modifications are certainly possible and it would be impossible to explicitly describe every conceivable aspect of the present invention. Therefore, the failure to describe any such aspect is also not intended to create any limitation to the present invention. The limitations of the present invention are defined exclusively in the following claims and nothing within this specification is intended to provide any further limitation thereto.

## Claims

1. A hydrophilic, water insoluble, carboxylic polymeric bioadhesive type material useful as a biolubricant, medicinal carrier medium or the like, said bioadhesive being manufactured according to the following process:

   charging a reactor continuously or in a single step with a solution comprising a non-aqueous solvent and a monomer charge dissolved therein, said monomer charge comprising carboxylic monomer having at least one activated carbon-to-carbon olefinic double bond, and at least one carboxyl group, wherein the amount of carboxyl containing monomer is in the range of about 5% to 100% by weight of the monomer charge and wherein the balance of the monomer charge comprises vinylidene comonomer;

   polymerizing the reagent system into a polymerization product in a closed vessel in an inert atmosphere under autogenous pressure or in an open vessel under reflux at atmospheric pressure,

   maintaining a temperature of polymerization in the range of about 0°C to about 100°C, wherein the solubility of the polymerization product causes the polymerization product to precipitate out of solution to define an average particle size of less than about 10 microns.

2. The bioadhesive of Claim 1 further comprising a crosslinking agent.

3. The bioadhesive of Claim 2 wherein the Brookfield mucilage viscosity of the polymerization product is greater than about 10,000 cps in the form of a 1% by weight aqueous mucilage at 20 rpm.

4. The bioadhesive of Claim 3 wherein the bioadhesive provides an adhesion of at least 50 dynes per square centimeter when measured between two pieces of freshly excised rabbit stomach tissue.

5. A process for manufacturing a bioadhesive, said process comprising the steps of:

   charging a reactor continuously or in a single step with a solution comprising a non-aqueous solvent and a monomer charge dissolved therein, said monomer charge comprising carboxylic monomer having at least one activated carbon-to-carbon olefinic double bond, and at least one carboxyl group, wherein the amount of carboxyl containing monomer is in the range of about 5% to 100% by weight of the monomer charge and wherein the balance of the monomer charge comprises vinylidene comonomer;

   polymerizing the reagent system into a polymerization product in a closed vessel in an inert atmosphere under autogenous pressure or in an open vessel under reflux at atmospheric pressure,

   maintaining a temperature of polymerization in the range of about 0 C to about 100 C, wherein the solubility of the polymerization product causes the polymerization product to precipitate out of solution to define an average particle size of less than about 10 microns.